# EUROPEAN PATENT APPLICATION

(11) **EP 1 004 328 A1**
(43) Date of publication of application: **31.05.2000**
(21) Application number: 99308713.9
(22) Date of filing: 02.11.1999
(51) Int. Cl.: A61M 25/00

(54) **Inflation indicators for balloon catheter**

(30) Priority: 27.11.1998 GB 9825881
(71) Applicant: Smiths Industries Public Limited Company, London, NW11 8DS (GB)
(72) Inventor: Parry, Andrew Martin, Dymchurch, Romney Marsh, Kent TN29 0TE (GB)
(74) Representative: Flint, Jonathan McNeill

(57) **Abstract**

An inflation indicator balloon 13 for a cuffed tracheal tube is moulded directly onto an and of an inflation line 10. The inflation line 10 is slipped on one end of a core pin 35 and a parison 60 is injection moulded in a first cavity 49, 50 with one end of the parison on top of the inflation line 10. The parison 60 is then blow moulded to the desired shape of the inflation balloon 13 in a second cavity 63, 64. The balloon 13 and line 10 are removed from the core pin 35 and a valve 14 is inserted in the end oposite the inflation line.

## Description

This invention relates to methods of manufacture of inflation indicators.

Cuffed medical tubes, such as tracheal tubes, are provided with some form of inflation indicator so that a clinician can determine whether the cuff is inflated or deflated when the tube is located in the body. The inflation indicator may be a flat balloon joined at one end with a small bore inflation line and at the other end with a connector and valve by which a syringe or similar device is connected to the inflation line. When the cuff of the tube is inflated, pressure is communicated to the inflation indicator, which is inflated to a more circular section. Conventionally, the indicator balloon is blow moulded and subsequently assembled onto and attached to the inflation line, the valve/connector and to its dust cap. Blow moulding a shape of this kind tends to lead to a relatively large amount of waste material and the assembly operations also add to the cost of the final product. It can also be a problem joining the balloon to the inflation line without leaking because the flat shape of the balloon does not lend itself to forming an effective seal around the cylindrical surface of the inflation line. Examples of inflation indicators are described in, for example, US 3810474, US 4018231, US 4130617, US 4178939, GB 2164565, GB 2174303 and GB 2213592.

It is an object of the present invention to provide an alternative inflation indicator and method of manufacture.

According to the present invention there is provided a method of manufacture of an inflation indicator, characterised in that the method comprises the steps of forming a parison of generally tubular shape about an end of an inflation line, then moulding the parison to the shape of an inflation balloon with the inflation line in situ such that the end of the inflation line is attached sealingly with one end of the balloon.

The inflation line is preferably fitted on one end of a core pin and the parison is preferably moulded in a first cavity on the core pin about an end of the inflation line. The parison is preferably moulded on the core pin to the shape of the inflatable balloon in a second cavity. The parison may be formed by injection moulding and be moulded to the shape of the inflation balloon by blow moulding. The balloon preferably has a generally flat shape. The method may include the subsequent step of joining a valve assembly in an end of the indicator opposite the inflation line. The parison may be formed with an integral dust cap.

A cuffed tracheostomy tube with an inflation indicator, and its method of manufacture, will now be described, by way of example, with reference to the accompanying drawings, in which:
- Figure 1: is a side elevation view of the tube;
- Figure 2: is an enlarged side elevation view of the inflation indicator;
- Figure 3: is an enlarged sectional view along the line III-III of Figure 2;
- Figure 4: is an enlarged transverse sectional view along the line IV-IV of Figure 2;
- Figure 5: is a sectional side elevation view of a first station of the machine used to preform the inflation indicator;
- Figure 6: is an enlarged sectional side elevation view of the pre-form produced by the machine shown in Figure 5; and
- Figure 7: is a sectional side elevation view of a second station of the machine used to form the inflation indicator.

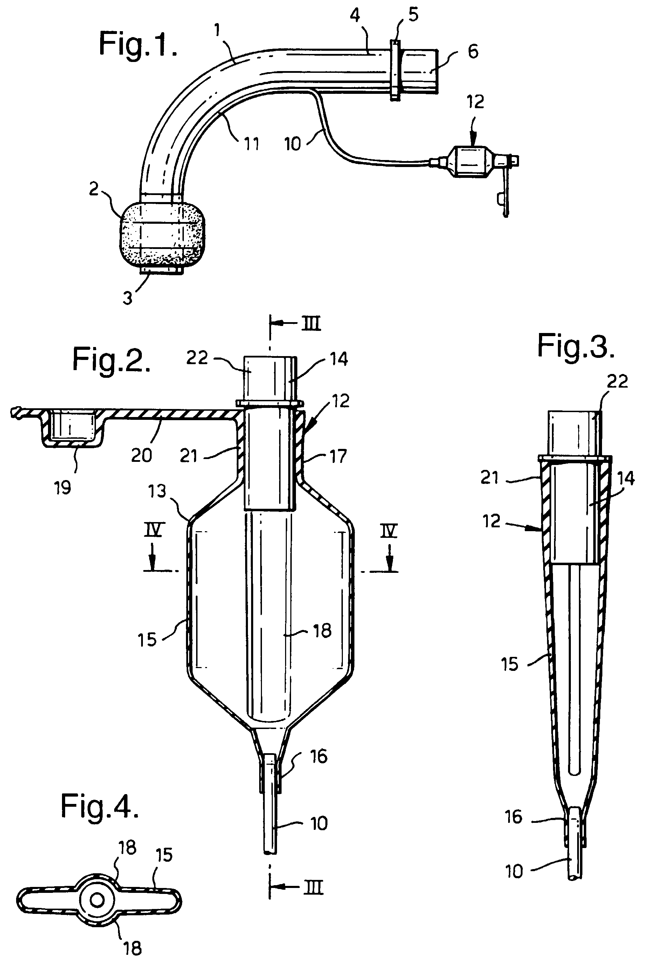

With reference first to Figures 1 to 4, the tracheostomy tube includes a curved, moulded shaft 1 of circular section with an inflatable cuff 2 surrounding the shaft close to the patient end 3. The machine end 4 of the tube has an integrally moulded flange 5 and connector 6. The cuff 2 is inflated and deflated by means of a small bore inflation line 10 secured in a channel 11 extending along the outside of the shaft 1 or via an extruded lumen within the wall of the tube. The patient end of the inflation line 10 extends within and opens into the interior of the cuff 2. The machine end of the inflation line 10 is provided with an inflation indicator 12.

The inflation indicator 12 includes a moulded balloon 13 and a separate valve/connector assembly 14. The balloon 13 includes an inflatable region 15, a patient end portion 16 joined with the inflation line 10, and a machine end portion 17 joined with the valve assembly 14. The inflation balloon 13 is of PVC and is moulded in a two-stage process of injection and blow moulding, which will be described in greater detail later. The inflatable region 15 is of generally rectangular shape with tapered ends and is substantially flat (in its uninflated state), with a raised rib 18 extending longitudinally in the centre of each side to ensure a continuous gas path through the indicator, even when a negative pressure is applied (as described in greater detail in GB 2213592). The balloon 13 is about 50mm long, with the inflatable region 15 being about 35mm long and 20mm wide. The wall thickness of the balloon 13 along the inflatable region 15 and the patient end 16 is about 0.5mm, whereas at the machine end, the wall is about 2mm thick. The balloon 13 is moulded with an integral dust cap 19 attached with the machine end of the indicator by a flexible web 20 about 25mm long.

The patient end portion 16 is in the form of a short collar of cylindrical shape, with an internal diameter of about 1.4mm and an external diameter of 2.4mm. The collar 16 is moulded about and bonded to the machine end of the inflation line 10, as will be described in greater detail later. At its machine end 17, the balloon 13 has a larger diameter sleeve 21, with an internal diameter of about 7mm. The valve assembly 14 is conventional and is bonded into the sleeve 21, the external end of the valve assembly having a female connector 22 shaped to allow the nose of a syringe to be inserted. The connector 22 is shaped to allow the nose of the syringe to contact and displace a valve element (not shown) from a valve seat within the assembly 14 so as to open a gas flow passage through the valve. At the same time, the nose of the syringe forms a seal with the connector 22.

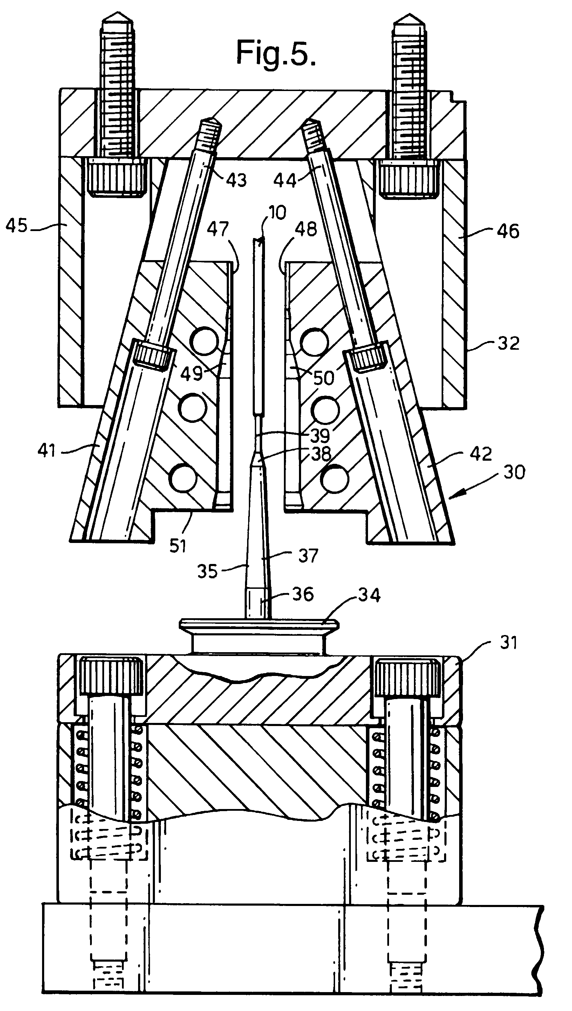

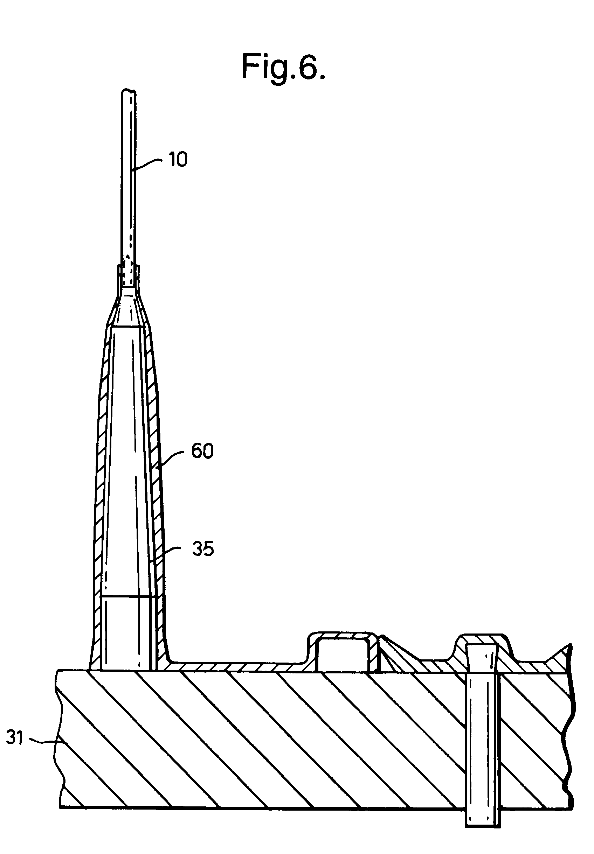

With reference now to Figures 5 to 7, the inflation indicator balloon 13 is made in two stages on a moulding machine 30 having a base assembly 31 and two different upper stations 32 and 33. As shown in Figure 5, the base assembly 31 comprises a base 34 and a vertically-extending, hollow and porous core pin 35, the external shape of which defines the internal shape of a preform of the indicator. More particularly, the core pin 35 has a lower section 36 of cylindrical shape and diameter of 7mm, an intermediate section 37, which tapers at a shallow angle to a reduced diameter to a more steeply tapered shoulder 38, which joins with a small diameter upper section 39. The upper section 39 receives the machine end of the inflation line 10 pushed onto the core pin 35 by a distance of about 4mm. The first upper station 32 is an injection moulding station and has two mould parts 41 and 42 that are slidable down and outwardly along angled pins 43 and 44 extending parallel to respective heel blocks 45 and 46. The mould parts 41 and 42 have respective opposed, vertical faces 47 and 48 shaped with recesses or cavities 49 and 50 to define a cavity for the external shape of the balloon preform 60 (Fig 6). More particularly, the cavities 49 and 50 conform substantially to the profile of the core pin 35 except that the spacing between the recesses and the core pin allows for a thicker layer of material along the intermediate section 37 than along the shoulder 38 and upper section 39. The lower surface 51 of one of the mould parts 41 is formed with a recess and the base 34 is formed with a projection (neither shown) which together define the dust cap 19 and web 20 of the balloon 13.

In operation, the upper station 32 is lowered about the base assembly 31 and the two mould parts 41 and 42 are closed around the core pin 35 with the inflation line 10 in place. Molten PVC is then injected into the cavity between the mould parts 41 and 42 and the core pin 35. The upper station 32 is then lifted and the mould parts 41 and 42 are separated, leaving a preform or parison 60 of the kind shown in Figure 6 on the base assembly 31, with an integral, moulded dust cap.

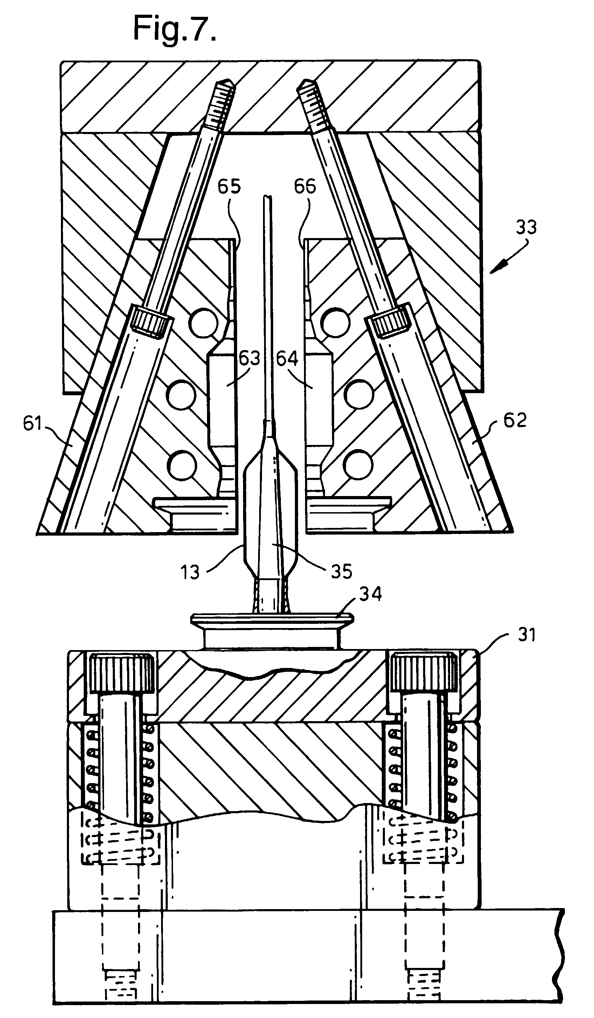

The base assembly 31 is then moved on to the second, blow station 33 (Figure 7), which also has two mould parts 61 and 62 with recesses or cavities 63 and 64 in their respective opposed vertical faces 65 and 66. These cavities 63 and 64 are shaped with the external profile of the final shape of the balloon 13, that is, they define the generally flat shape of the indicator balloon.

The blow station 33 is lowered and the mould parts 61 and 62 are closed about the core pin 35 and preform 60. The mould parts 61 and 62 are heated and pressure is applied via the core pin 35 to the inside of the preform 60 so that this is blown outwardly to fill the shape of the cavities 63 and 64. The upper station 33 is then raised and the mould parts 61 and 62 are separated allowing the balloon 13 in its final form to be removed from the core pin 35. The indicator 12 is completed by inserting the valve assembly 14 into the sleeve 21 and bonding this in position such as by welding or adhesive. It may be necessary in some cases for the blow moulding stage to be carried out in two parts. The parison is first blow moulded to an intermediate shape at a first blow station and is then moved to a second blow station where it is blow moulded to its final shape. This helps ensure a more constant wall thickness over the indicator balloon.

It can be seen that, because the patient end of the balloon is moulded about the inflation line, there can be a perfect seal with the inflation line, which cannot always be guaranteed when previous assembly techniques are used. The manufacturing method described enables the number of manual assembly operations required to be greatly reduced. Assembly is also reduced because the dust cap can be moulded integrally with the balloon, rather than being made separately and subsequently assembled. The amount of waste material produced by the injection blow moulding technique described can be less than previous blow moulding techniques. The two stage process of injection moulding and blow moulding also enables more accurate control of wall thickness of the final product.

## Claims

1. A method of manufacture of an inflation indicator (12), characterised in that the method comprises the steps of forming a parison (60) of generally tubular shape about an end of an inflation line (10), then moulding the parison (60) to the shape of an inflation balloon (13) with the inflation line in situ such that the end of the inflation line is attached sealingly with one end of the balloon.

2. A method according to Claim 2, characterised in that the inflation line (10) is fitted on one end of a core pin (35) and the parison (60) is moulded in a first cavity (49, 50) on the core pin (35) about an end of the inflation line (10).

3. A method according to Claim 2, characterised in that the parison (60) is moulded on the core pin (35) to the shape of the inflation balloon (13) in a second cavity (63, 64).

4. A method according to any one of the preceding claims, characterised in that the parison (60) is formed by injection moulding and that the parison is moulded to the shape of the inflation balloon (13) by blow moulding.

5. A method according to any one of the preceding claims, characterised in that the balloon (13) has a generally flat shape.

6. A method according to any one of the preceding claims, characterised in that the method includes a subsequent step of joining a valve assembly (14) in an end of the indicator (12) opposite the inflation line (10).

7. A method according to any one of the preceding claims, characterised in that the parison (60) is formed with an integral dust cap (19).
